(19) Europäisches Patentamt / European Patent Office / Office européen des brevets

(11) **EP 4 241 779 A1**

(12) **EUROPEAN PATENT APPLICATION**

(43) Date of publication:
**13.09.2023 Bulletin 2023/37**

(21) Application number: **22315052.5**

(22) Date of filing: **09.03.2022**

(51) International Patent Classification (IPC):
**A61K 36/73** (2006.01)    **A61P 3/10** (2006.01)
**A23L 2/02** (2006.01)    **A61K 31/352** (2006.01)
**A61P 39/06** (2006.01)

(52) Cooperative Patent Classification (CPC):
**A61P 3/10; A23L 2/02; A23L 2/52; A23L 2/58;
A61K 31/352; A61K 36/73; A61P 39/06;
A61K 2236/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Diana Food
35560 Val Couesnon (FR)**
• **Diana
56250 Saint-Nolf (FR)**

(72) Inventors:
• **LASTIQUE, Bénédicte
35000 RENNES (FR)**
• **VILLA RODRIGUEZ, José
35000 RENNES (FR)**
• **GUYONNET, Denis
92300 LEVALLOIS-PERRET (FR)**

(74) Representative: **De Crignis, Margot Gabrielle
Diana
1, allée Ermengarde d'Anjou
35000 Rennes (FR)**

(54) **ARONIA EXTRACTS AND USES THEREOF**

(57)    The invention relates to 1) an aronia extract comprising proanthocyanidins (PACs) and anthocyaniris, wherein the polyphenol content is at least 55 dry wt.%, the PACs content is at least 19 dry wt.%, and the PACs:anthocyanins ratio is at least 1.5; 2) a process for obtaining the aronia extract and the extract obtainable by said process; 3) a food, nutraceutical, or cosmetic composition comprising, or consisting of, said aronia extract and its use for preventing, limiting, or reducing the production of oxidative stress due to the exposure to pollutants, aging and/or exercise; 4) a pharmaceutical composition comprising said aronia extract and 5) such a pharmaceutical composition for use in the treatment of a disorder related to cellular oxidative stress in a subject in need thereof.

FIG. 1

**Description**

**Field of the invention**

The invention is in the field of natural extracts and their uses as ingredient for food, nutraceutical, or pharmaceutical products. In particular, the invention relates to aronia extracts having a high total polyphenolic content, a high proanthocyanidins content and a specific proanthocyanidins to anthocyanins ratio.

**Background**

In the past few decades, great attention has been focused to natural products sources of antioxidants and their beneficial effects to human health. According to the "oxidative stress" theory, an imbalance between reactive oxygen species (ROS) and antioxidants causes a loss of redox signaling and damage to DNA, proteins, and lipids, whose accumulation leads to aging and various degenerative and chronic diseases (Holmstrom and Finkel 2014). Hence, an excess production of reactive species due to a stressor exposure (pollutants, exercise, injury, aging) is involved in the initiation and progression of several diseases. Under normal conditions, ROS are neutralized by antioxidant defense systems, some are synthetized in vivo (endogenous cellular antioxidant defenses including SOD, catalase, glutathione) and other come from our diet (exogenous phytochemicals or dietary antioxidants like vitamins C and E). Since higher fruit, vegetable and legume intake is associated with lower mortality in populations (Fraga et al. 2019), the importance of higher plant product consumption has received increased attention from the consumer. With more than 8000 known polyphenolic compounds being identified in the plant kingdom, these plant secondary metabolites are the most abundant dietary antioxidants (Pandey and Rizvi 2009).

Berry bushes such as red, purple, and black chokeberry (e.g. Aronia melanocarpa, Aronia prunifolia, and Aronia arbutifolia), are coming into the focus of researchers and the public, for health-promoting and chronic disease-preventing properties, particularly due to their great antioxidative activity (Borowska and Brzóska 2016). Compared to other natural products, aronia fruits are some of the richest sources of polyphenolic compounds, with a high content in phenolic acids (mostly chlorogenic and neochlorogenic acids) and flavonoids. Flavonoids present in aronia can be divided into four subgroups: anthocyanins, flavonols, flavan-3-ols (or flavanols) and proanthocyanidins. Berries of aronia are known for their richness in anthocyanins (mostly cyanidin-3-galactoside and cyanidin-3-arabinoside) which give the dark color and can be used as food colorants. Aronia flavonols consist mainly in five quercetin derivatives (quercetin-3-O-galactoside, quercetin-3-O-glucoside, quercetin-3-O-rutinoside, quercetine-3-O-robinobioside and quercetin-3-O-vicianoside). Flavan-3-ols exist as monomeric forms (epicatechin), or oligomeric and polymeric forms (composed of epicatechin units) which are referred to as proanthocyanidins (PACs) (or called procyanidins when they consist exclusively of epicatechin units, like in Aronia where they are type B procyanidins). The degree of proanthocyanidin polymerization is a characteristic feature of aronia. Moreover, aronia contains active compounds with antioxidant properties other than polyphenols, such as antioxidative vitamins (C and E), carotenoids, and minerals such as Zn, Cu, Mo and Se.

WO2021122789A1 discloses an antioxidant composition containing anthocyanins or extracts having the defined anthocyanins. US20120128800A1 discloses a dietary supplement composition comprising an Aronia melanocarpa extract containing at least 20% of anthocyanins by dry weight.

Even though several publications have pointed out aronia extracts as good candidates for antioxidant effects and for the treatment of prevention of related disorders, there is still a need for new extracts and enriched fractions thereof with improved efficacy in order to offer even more relevant products to the food, nutraceutical and pharmaceutical industries.

This need is addressed by the present invention with the provision of new aronia extract, which demonstrates a particularly high antioxidant efficacy, and which are therefore particularly suitable for food, nutraceutical, cosmetic and pharmaceutical application. For the first time, this efficacy has been assessed in innovative cell-based assays for testing direct antioxidant activities.

**Summary of the invention**

A first object of the present invention related to an aronia extract comprising proanthocyanidins (PACs) and anthocyanins, wherein:

-    the polyphenol content is at least 55 dry wt.%, preferably at least 60 dry wt.%,

-    the PACs content is at least 19 dry wt.%, preferably at least 20 dry wt.%,

-    the PACs:anthocyanins ratio is at least 1.5, and

wherein the polyphenol content can be measured by the Folin Ciocalteu (epicatechin equivalent), the proanthocyanidin content can be measured by the BL-DMAC method (procyanidin A2 equivalent), and the anthocyanin content can be measured by spectrophotometry (cyanidin 3-O-glu Cl equivalent).

In a particular embodiment, the ratio of PACs to anthocyanins of the aronia extract of the invention is comprised between 1.5 and 4.5, preferably between 1.5 and 3.5, more preferably between 1.5 and 2.5, and even more preferably is of about 2.

In another embodiment, the anthocyanins content of the aronia extract of the invention is at least 10 dry wt.%, preferably between 10 and 20 dry wt.%, more preferably between 10 and 15 dry wt.%.

In a preferred embodiment, the aronia extract of the invention is selected from the group consisting of an *Aronia arbutifolia* extract, an *Aronia melanocarpa* extract and an *Aronia prunifolia* extract. More preferably, the aronia extract is an extract obtained from the fruits of *Aronia melanocarpa.*

According to a particular embodiment, the aronia extract of the invention is in a dry powder form. In this embodiment, the extract can have the following color parameters, when measured at 0.04 g/L of anthocyanin in a buffer at a pH value of 3:

- L* (brightness) between 65 and 70,

- a* (red/green coordinate) of less than 55, preferably between 45 and 55, more preferably between 50 and 54,

- b* (yellow/blue coordinate) of less than 20, preferably of less than 15, more preferably between 10 and 15.

Another object of the invention relates to a process for obtaining the aronia extract according to the first object, the process comprising the steps of:

- selecting a concentrated juice of aronia fruits with an anthocyanin concentration (measured as cyanidin equivalent) of at least 0.5% at 65 Brix,

- diluting the concentrated juice to about 25 Brix with osmosed water to obtain a diluted juice,

- contacting, in a chromatographic column, said diluted juice with a macroporous, crosslinked, aliphatic polymer adsorbent resin, wherein the resin has a surface area of at least 300 $m^2$/g, an average pore diameter of about 550 Angstrom, a total pore volume of about 0.5 mL/mL, a water retention capacity of 61 to 69%, said resin being conditioned at a pH equal to or of less than 3.5 with acidified water,

- washing with acidified water,

- eluting polyphenols using an acidified hydro-ethanolic solution to obtain an elution solution,

- optionally, concentrating the elution solution to a dry matter content from 35 to 55% w/w, to obtain a concentrated extract,

- optionally, drying and grinding said concentrated extract to obtain a dry extract,

wherein said acidified water is obtained by mixing water with 0.03% (volume/volume) of a hydroxide sulfate solution at a concentration of 96%.

In a preferred embodiment, the concentrated juice of Aronia fruits of the process for obtaining the aronia extract according to the invention is a concentrated fruit juice obtained from *Aronia melanocarpa.*

The invention also relates to an aronia extract obtainable by said process for obtaining the aronia extract.

Another object of the present invention relates to a food, nutraceutical or cosmetic composition comprising, or consisting of, the aronia extract according to the first object.

Another object of the invention relates to the use of such a composition, for preventing, limiting, or reducing oxidative stress due to the exposure to pollutants, aging and/or exercise.

According to another object, the invention also relates to a pharmaceutical composition comprising the aronia extract according to the first object. In another object, the invention relates to such a pharmaceutical composition for use in the treatment of a disorder related to cellular oxidative stress in a subject in need thereof, such as gut barrier dysfunction, intestinal inflammation, inflammatory bowel diseases, irritable bowel disease, insulin resistance, type 2 diabetes mellitus, metabolic syndrome, cardiovascular disorders, and neurodegenerative disorders.

**Brief description of the figures**

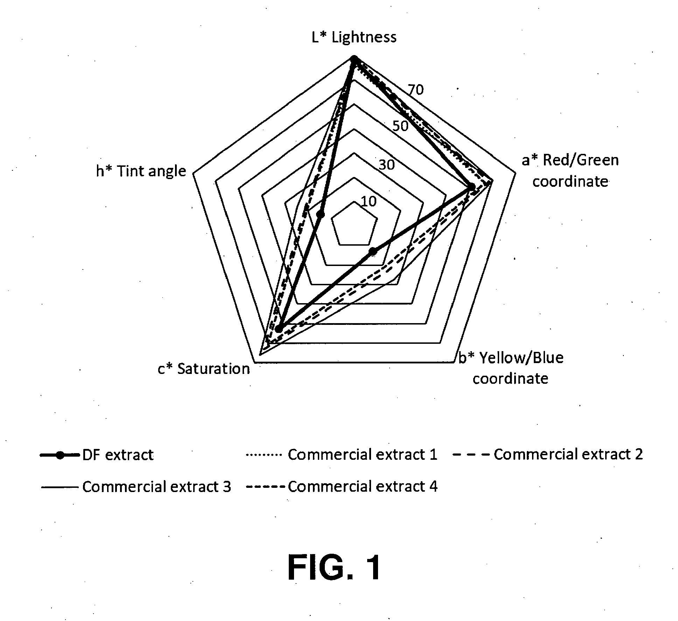

Figure 1: Color values (L*a*b*c*h) of an aronia extract according to the invention and three commercially available aronia extracts.

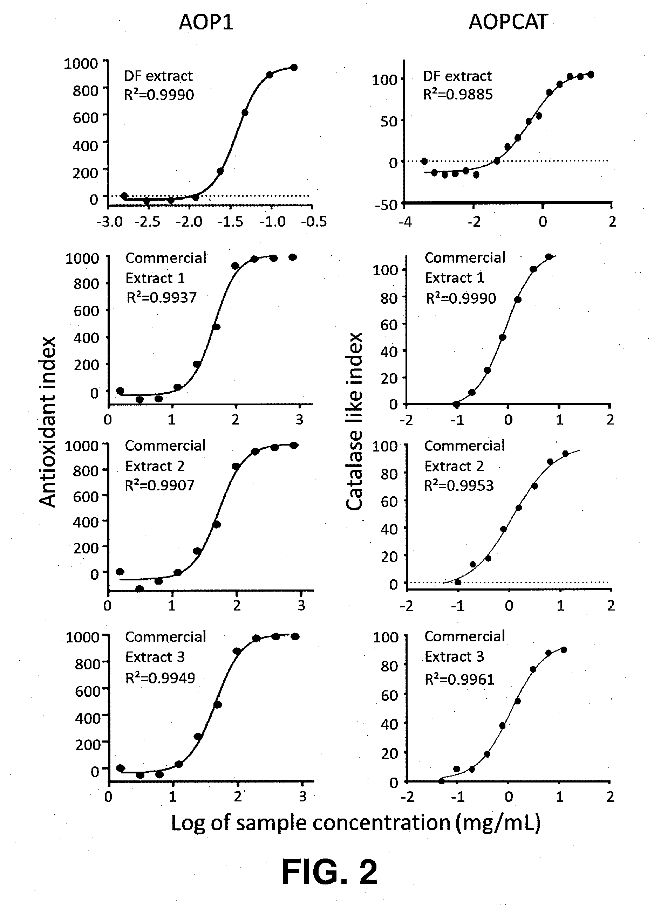

Figure 2: Dose-response curves of an aronia extract according to the invention and three commercially available aronia extracts, as measured in AOP1 and AOPCAT assays in HepG2 cells.

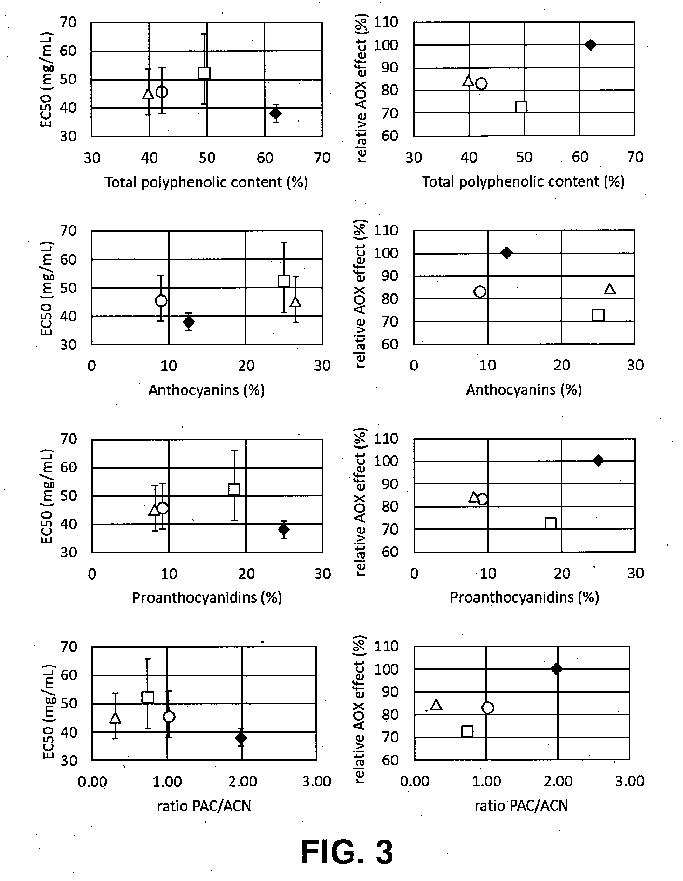

Figure 3: EC50 (mg/mL, left column) and relative antioxidant effect (relative AOX effect in %, right column) as measured in HepG2 cells during the AOP1 assay, expressed as a function of total polyphenolic content (dry weight %), anthocyanin content (dry weight %), proanthocyanidins content (dry weight %) and of the ratio PACs/anthocyanins (ratio PAC/ACN). Black diamonds: DF extract; Open circles: commercial extract 1; Open squares: commercial extract 2; Open triangles: commercial extract 3; Bars represent the 95% confident interval of calculated EC50.

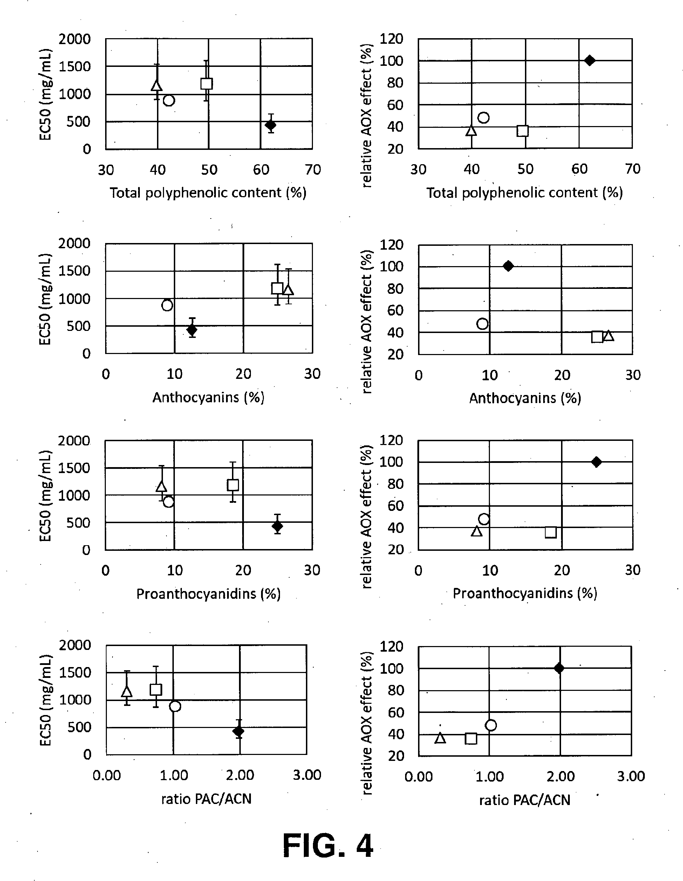

Figure 4: EC50 (mg/mL, left column) and relative antioxidant effect (relative AOX effect in %, right column) as measured in HepG2 cells during the AOPCAT assay, expressed as a function of total polyphenolic content (dry weight %), anthocyanin content (dry weight %), proanthocyanidins content (dry weight %) and of the ratio PACs/anthocyanins (ratio PAC/ACN). Black diamonds: DF extract; Open circles: commercial extract 1; Open squares: commercial extract 2; Open triangles: commercial extract 3; Bars represent the 95% confident interval of calculated EC50.

**Detailed description**

Definitions

In the context of the invention, "polyphenols" relates to molecules formed by more than one phenol unit. Flavonoids are the most commonly occurring polyphenols, and are, e.g., flavonols, flavanols, catechins, flavanones, anthocyanidins, isoflavonoids.

In the context of the invention, "proanthocyanidin" (PAC) or "proanthocyanidins" (PACs) or "condensed tannins" corresponds to oligomers or polymers of flavanols. The term "oligomer" includes flavanol dimers and trimers, and the term "polymer" includes flavanol multimers having a degree of polymerization (DP) of at least 4.

In the context of the invention, "anthocyanins" or "anthocyans" or "anthocyanosides" means glycosides of anthocyanidins, a subclass of flavonoids. The saccharide moiety of anthocyanins can be a monosaccharide (glucose, galactose, rhamnose), a disaccharide (rutinose, composed of a glucose linked to a rhamnose, xyloglucose) or at times a trisaccharide. Anthocyanins are water soluble plant pigments responsible for red, blue and purple colors found in many plants, including flowers and fruits.

In the context of the invention, "dry wt.%" refers to the percentage by weight of a compound relative to the total dry weight of an extract according to the invention unless specifically stated otherwise. For instance, an extract with a polyphenols content of 55 dry wt.% means that there is 55 g of polyphenols in 100 g of dry total extract.

As used herein, "treatment" of a disorder includes the therapy or the prophylaxis of the disorder, or the prevention or delay in the onset of the disorder, or reduction of symptoms provoked by the disorder. The term treatment includes in particular the control of disease progression and associated symptoms.

As used herein, a "subject" refers to an individual receiving treatment. In one aspect, a subject can be a mammal. In another aspect, a subject can be a human. In another aspect, the subject can be a domesticated animal or livestock.

The "antioxidant properties" of bioactive compounds refer to several definitions or potential cellular mechanisms. Antioxidants, as agents that donate electrons or hydrogen atoms to oxidants, preventing them from oxidizing other molecules, exert direct effects including radical scavenging activity, suppression of the formation of reactive species, restoration of antioxidant enzyme and inhibition of prooxidant enzymes, or chelation of metal ions. Besides, biological effects elicited by indirect antioxidant properties of phytochemicals can be attributed to their ability to induce the redox sensitive transcription factor, Nuclear erythroid 2- related factor 2 (Nrf2) (Forman, Davies, and Ursini 2014).

For the first time, the inventors have assessed the direct antioxidant effects of aronia extracts in innovative cell-based assays. Surprisingly, they have demonstrated that these direct effects is related to high polyphenol content combined with high PACs content (instead of a high anthocyanidin content), and also to the ratio of PACs to anthocyanins.

Aronia extracts

The aronia extracts according to the present invention are obtained from plants of the genus Aronia. This genus encompasses shrubs known as chokeberries and belongs to the Rosaceae family. The main species of Aronia genus are Aronia arbutifolia (red aronia), Aronia melanocarpa (black chokeberry) or Aronia prunifolia (purple aronia). In a preferred embodiment, the aronia extract of the invention is an extract from Aronia melanocarpa.

*Polyphenols*

The aronia extract according to the present invention is rich in polyphenols. Methods for identifying and quantifying phenolics in extracts or compositions are known in the art and include, but are not limited to, for example, direct spectroscopy at 280 nm; indirect spectroscopy using, e.g., art recognized and commercially available reagents and assays, e.g., Vanillan assay, Folin-Denis assay, Folin-Ciocalteu assay, Prussian Blue assay, Bate-Smith assay, and Porter assay; and liquid chromatography, e.g., using ultraviolet, fluorescence, mass spectroscopy, and nuclear magnetic resonance (NMR). Phenolics detection techniques are also disclosed in the literature (see, e.g., (Fereidoon and Naczk 1995)).

The polyphenol content of the aronia extract according to the invention is of at least 55 dry wt.%, as it can be measured by the Folin Ciocalteu method and expressed as epicatechin equivalent. For example, extract according to the invention can contain at least 56 dry wt.%, at least 57 dry wt.%, at least 58 dry wt.%, at least 59 dry wt.%, at least 60 dry wt.%, at least 61 dry wt.% or at least 62 dry wt.% of total polyphenols, as it can be measured by the Folin Ciocalteu method and expressed as epicatechin equivalent. More preferably, the polyphenol content of the extract according to the invention is of at least 60 dry wt.%, as it can be measured by the Folin Ciocalteu method (epicatechin equivalent).

*Anthocyanins*

The aronia extract of the invention comprises anthocyanins. Anthocyanin content can be measured by HPLC, HPLC-MS, European Pharmacopoeia or adaptation thereof, such as spectrophotometry at 528 nm and expression of the anthocyanin content as cyanidin-3-O-glucoside chloride equivalent.

In an embodiment, the anthocyanins content is at least 10 dry wt.%, preferably between 10 and 20 dry wt.%, more preferably between 10 and 15 dry wt.%, as it can be measured by spectrophotometry at 528 nm expressed as cyanidin-3-O-glucoside chloride equivalent. For example, the aronia extract of the invention can contain between 10 dry wt.% and 20 dry wt.%, i.e. about 10.5 dry wt.%, about 11 dry wt.%, about 11.5 dry wt.%, about 12 dry wt.%, about 12.5 dry wt.%, about 13 dry wt.%, about 13.5 dry wt.%, about 14 dry wt.%, about 14.5 dry wt.%, about 15 dry wt.%, about 15.5 dry wt.%, about 16 dry wt.%, about 16.5 dry wt.%, about 17 dry wt.%, about 17.5 dry wt.%, about 18 dry wt.%, about 18.5 dry wt.%, about 19 dry wt.%, about 19.5 dry wt.% or about 20 dry wt.% of anthocyanins as it can be measured by spectrophotometry at 528 nm expressed as cyanidin-3-O-glucoside chloride equivalent.

Surprisingly, the anthocyanin content was not positively correlated to the direct antioxidant efficacy of the aronia extract (cf. Example 3).

*PACs*

The aronia extract of the invention comprises proanthocyanidins. The PACs content in an extract or a composition can be assessed using DMAC, BL-DMAC assay's (Prior et al. 2010) or adaptation thereof, such as BL-DMAC expressed as procyanidin A2 equivalent, phloroglucinolysis, the European Pharmacopeia or adaptation thereof.

The proanthocyanidin content of the aronia extract according to the invention is of at least 19 dry wt.%, as it can be measured by the BL-DMAC method and expressed as procyanidin A2 equivalent. In a preferred embodiment, the proanthocyanidin content of the extract according to the invention is of at least 20 dry wt.%, as it can be measured by the BL-DMAC method (procyanidin A2 equivalent). More preferably, the proanthocyanidin content of the extract according to the invention is of at least 21 dry wt.%, as it can be measured by the BL-DMAC method (procyanidin A2 equivalent).

Surprisingly, the PACs content was strongly positively correlated to the antioxidant efficacy of the aronia extract when direct antioxidant effect was measured, whereas the anthocyanins content was not (cf. Example 3).

*Ratio proanthocyanidins/anthocyanins*

The inventors have found that the ratio PACs/anthocyanins was the strongest component of the antioxidant effect observed in the aronia extracts (cf. Example 3). The aronia extract of the invention is characterized by a PACs:anthocyanins ratio of at least 1.5. In a preferred embodiment, the PACs:anthocyanins ratio is comprised between 1.5 and 4.5, preferably between 1.5 and 3.5, more preferably between 1.5 and 2.5, and even more preferably is of about 2. Surprisingly, this feature has been shown to be strongly positively correlated to the antioxidant effect of the aronia extract (cf. Examples, AOPCAT model and Fig. 4).

*Powder & colour*

The extract of the invention may be provided in various forms, in particular in solution form, or in dry form. Advantageously, the aronia extract of the invention is in a dry powder form. Such a powdered extract is advantageous because it reduces the volume for transportation. Also, it is more convenient to process further the powder within a matrix, e.g. to obtain a food, nutraceutical, cosmetic or pharmaceutic composition, such as e.g. tablets, capsules, granules and orodispersible solutions. Known methods can be used to dry the extract of the invention, such as evaporation, vacuum drying, spray drying, roller drying, freeze-drying, etc. In solid or liquid form, it can also make it possible to formulate gel, creams, soaps for topical application, and to formulate beverages that are reconstituted by dilution or are ready to use.

Color can be defined by several methods, such as Hunter L, a, b scale or the CIE 1976 L*a*b scale. According to the CIE (Commission Internationale de l'Eclairage), the L*a*b* color space is modeled after a color-opponent theory stating that two colors cannot be red and green ate the same time or yellow and blue at the same time. L* indicates the brightness, a* is the red/green coordinate, and b* is the yellow/blue coordinate.

In a preferred embodiment, the extract according to the invention has the following color parameters, when measured at 0.04 g/L of anthocyanin in a buffer at a pH value of 3 (Hunter L, a, b scale):

- L* (brightness) between 65 and 70,

- a* (red/green coordinate) of less than 55, preferably between 45 and 55, more preferably between 50 and 54,

- b* (yellow/blue coordinate) of less than 20, preferably of less than 15, more preferably between 10 and 15.

According to a preferred embodiment, the powdered aronia extract according to the invention can have the following additional color parameters, when measured at 0.04 g/L of anthocyanin in a buffer at a pH value of 3 (Hunter L, a, b scale):

- c* (saturation) of less than 60, preferably of less than 55, more preferably between 50 and 55, and

- h* (tint angle) of less than 20, preferably of less than 18, more preferably between 10 and 18

Process for obtaining an aronia extract

Another object of the invention related to a process for obtaining an aronia extract as described above.

In an embodiment, the extract according to the present invention is obtained by phenolic extraction from a plant or part of a plant of *Aronia* genus. A part of a plant means the leaf or flowers or fruits, in a solid or liquid form (solid or liquid substrate).

In a preferred embodiment, the extract of the invention is obtained by phenolic extraction from a liquid substrate, such as a juice or a concentrated juice of plants or part of a plant of *Aronia* genus, preferably a juice of fruits (berries) from a plant of *Aronia* genus, more preferably a concentrated juice of fruits (berries) from a plant of *Aronia* genus.

In a very preferred embodiment, the extract according to the present invention is obtained by phenolic extraction from a concentrated juice of fruits of *Aronia melanocarpa,* and more preferably from a concentrated juice of fruits of *Aronia melanocarpa* comprising at least 0.5% (w/w) of anthocyanins at 65 Brix (cyanidin-3-O-glucoside chloride equivalent).

The preferred method for extracting polyphenols is chromatography extraction. In this embodiment, a solid substrate is diluted to become liquid, and a liquid substrate can advantageously be diluted. In a preferred embodiment, the substrate is diluted until a Brix value from about 20 to about 30, such as about 20°Brix, about 21°Brix, about 22°Brix, about 23°Brix, about 24°Brix, about 25°Brix, about 26°Brix, about 27°Brix, about 28°Brix, about 29°Brix or about 30°Brix, is reached. More preferably, a concentrated juice of fruits of aronia comprising at least 0.5% (w/w) of anthocyanins at 65° Brix (cyanidin -3-O-glucoside chloride equivalent) is diluted until reaching a Brix of about 25. The dilution can be made with known solutions, such as purified water.

The diluted substrate is then loaded in the chromatography column. The preferred resin for the method of the invention is Amberlite™ XAD-7HP resin (distributed by The Dow Chemical). XAD-7HP is a commercially available macroreticular aliphatic crosslinked polymer ester resin that consists of white translucent beads that have a high surface area of at least 300 m$^2$/g, i.e. of about 500 m$^2$/g, an average pore diameter of about 550 Angstroms, a total pore volume of about 0.5 mL/mL, a water retention capacity of 61 to 69%, a particle diameter of 430 to 690 μm, less than 7.0% of particles with a size under 300 μm, less than 8.0% of particles having a size above 1180 μm and a particle density from 1.06 to 1.08 g/mL.

More preferably, the resin is first conditioned with purified water. Purified water includes osmosed water, and preferably acidified osmosed water. Osmosed water can be acidified by adding an acid solution to osmosed water. In a preferred embodiment, acidified water is prepared by adding $H_2SO_4$ solution (about 96% v/v) to osmosed water to a final concentration of about 0.03% (v/v). In a preferred embodiment, the resin is conditioned at a pH equal to or of less than 3.5. In a more preferred embodiment, Amberlite™ XAD-7HP resin is conditioned at a pH equal to or of less than 3.5 with acidified water prepared by adding $H_2SO_4$ solution (96% v/v) to osmosed water to a final concentration of 0.03% (v/v).

The amount of resin used can be adapted and depend upon the scale of the process and/or the volume or capacity of the column.

The time and conditions sufficient for phenolic compounds present in the composition to bind the resin include those times and conditions under which the desirable quantity of phenolics present in the composition bind to the resin.

The substrate loaded in the chromatography resin can then be washed to eliminate sugars and some acids. Washing solutions that can be used in the process of the invention can include water-based solutions, such as pure water or solutions containing water and another compound, such as a solvent or an acid. In a preferred embodiment, purified water is used, and preferably acidified water such as described above. The washing step can be performed by the addition of 1 to 5 BV (Bed Volume) of acidified water to the column, such as 1 BV, 2 BV, 3 BV, 4 BV or 5 BV of acidified water. Preferably, the washing step is performed by the addition of 2 to 3 BV of acidified water.

The substrate loaded in the chromatography column can then be eluted to recover polyphenols that have bound to the resin. Elution solutions that can be used in the process of the invention can include water-based solutions containing one or more solvents at any concentration that will increase the release of polyphenols from the resin. In a preferred embodiment, the elution solution consists of an acidified hydro-ethanolic solution, such as a solution comprising from 1% to 99% v/v of ethanol and 99% to 1% of acidified water. A preferred acidified hydro-ethanolic solution comprises from about 30% to about 70% of ethanol and from about 70% to about 30% of acidified water, e.g. about 30% of ethanol and about 70% of acidified water, about 40% of ethanol and about 60% of acidified water, about 50% of ethanol and about 50% of acidified water, about 60% of ethanol and about 40% of acidified water, or about 70% of ethanol and about 30% of acidified water.

The elution flow is collected. The elution flow is an aronia extract comprising proanthocyanidins and anthocyanins according to an object of the present invention.

The elution flow can optionally be further processed.

For instance, solvent of the elution solution can be partially or totally evaporated. In a preferred embodiment, the elution flow is concentrated by evaporation, until a dry matter content from 35 to 55 % w/w is reached, preferably from 40 to 50 % w/w, such as about 35% w/w, about 36% w/w, about 37% w/w, about 38% w/w, about 39% w/w, about 40% w/w, about 41% w/w, about 42% w/w, about 43% w/w, about 44% w/w, about 45% w/w, about 46% w/w, about 47% w/w, about 48% w/w, about 49% w/w, about 50% w/w, about 51% w/w, about 52% w/w, about 53% w/w, about 54% w/w or about 55% w/w. Evaporation can be implemented at a temperature from 60 °C to 75°C, preferably at a temperature from 68°C to 72°C.

The concentrated elution flow can be dried, i.e., further concentrated until a higher dry matter content is reached, to obtain a dry extract. Suitable concentration methods include, but are not limited to, membrane concentration, heat concentration, vacuum (reduced pressure) concentration, and freeze concentration. In a preferred embodiment, the concentrated elution flow is dried by vacuum drying.

In a preferred embodiment, the dry extract is grinded to make a powder. The powder can advantageously be sieved. The mesh size for sieving can be from about 250 $\mu$m to about 1000 $\mu$m, preferably of about 500 $\mu$m.

According to a preferred embodiment, the dry powdered extract is packaged. This embodiment is advantageous for selling the extract as an ingredient, for instance as an ingredient for food, nutraceutical, cosmetic or pharmaceutic products.

Product by process

Another object of the invention is the aronia extract as obtained by the process of the invention described above. This aronia extract shares the features of the aronia extract as described above. In particular, the aronia extract has a polyphenol content of at least 55 dry wt.%, preferably at least 60 dry wt.%, a PACs content is at least 19 dry wt.%, preferably at least 20 dry wt.%, and the ratio of PACs to anthocyanins is at least 1.5, wherein the polyphenol content can be measured by the Folin-Ciocalteu (epicatechin equivalent), the proanthocyanidin content can be measured by the BL-DMAC method (procyanidin A2 equivalent), and the anthocyanin content can be measured by spectrophotometry (cyanidin 3-O-glucoside chloride equivalent).

Composition and uses thereof

According to another object, the invention relates to compositions comprising the aronia extract as described above.

It includes compositions which do not comprise any other compound than those of the aronia extract according to the invention. Hence, in an embodiment, the invention related to compositions consisting of the aronia extract according to the invention. Such compositions can be advantageous because the polyphenols are not diluted with other compounds and could be more effective as an antioxidant. It is also an interesting product for the manufacturers of food, nutraceutical or cosmetic compositions or products, who can use the aronia extract according to their own recipes.

In another embodiment, the composition comprises the aronia extract according to the invention with at least one other compound. For example, a composition according to the invention can comprise an aronia extract as described above and at least one additional compound, such as at least one additional compound selected from the group consisting of sweeteners, preservatives, flavoring agents, thickeners, coatings, isotonic agents, absorption delaying agents, binders, adhesives, lubricants, disintegrants, coloring agents, absorbents, detergents, emulsifying gents, antioxidants, vitamins, minerals, proteins, fats, carbohydrates, and also food matrix such as fruits, vegetables or meat preparations, or a combination thereof. Consequently, in a preferred embodiment, the composition comprising the aronia extract is a food, nutraceutical or cosmetic composition, depending on the additional compound(s) used in the composition.

A composition consisting of, or comprising, the aronia extract according to the present invention, has shown to be particularly effective for preventing, limiting, or reducing the intracellular oxidative stress in cells (see Example 3). In particular, it has shown superior effects compared to commercially available extracts, in a liver cell model. Hence, a composition consisting of, or comprising, the aronia extract according to the present invention is useful for preventing, limiting, or reducing the oxidative stress due to the exposure to pollutants, aging and/or exercise.

Because of the unexpected direct antioxidant effect of the aronia extract according to the present invention, other objects of the invention relate to a pharmaceutical composition comprising the aronia extract according to the invention and to such a pharmaceutical composition for use in the treatment of a disorder related to cellular oxidative stress in a subject in need thereof. Disorders related to cellular oxidative stress are well known in the art and there is a considerable literature that deals with this. In a preferred embodiment, the disorder related to cellular oxidative stress is selected from the group consisting of gut barrier dysfunction, intestinal inflammation, inflammatory bowel diseases, irritable bowel disease, insulin resistance, type 2 diabetes mellitus, metabolic syndrome, cardiovascular disorders, and neurodegenerative disorders.

A pharmaceutical composition comprising the aronia extract according to the invention typically comprises one or several pharmaceutically acceptable carriers or excipients. The dosage, frequency and mode of administration of the pharmaceutical composition of the invention and the duration of the therapy depends on the stage of the disease being treated, the age and condition of the patient, and how the patient responds to the treatment. Additionally, pharmacogenomic (the effect of genotype on the pharmacokinetic, pharmacodynamic or efficacy profile of a therapeutic) information about a particular patient may affect the dosage used.

Possible pharmaceutical compositions include those suitable for oral or topical (including transdermal, buccal and sublingual) administration.

More commonly these pharmaceutical compositions are prescribed to the patient in "patient packs" containing a number of dosing units or other means for administration of metered unit doses for use during a distinct treatment period in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in traditional prescriptions. The inclusion of a package insert has been shown to improve patient compliance with the physician's instructions. Thus, the invention further includes a pharmaceutical composition, as herein before described, in combination with packaging material suitable for said composition. In such a patient pack the intended use of a formulation for the combination treatment can be inferred by instructions, facilities, provisions, adaptations and/or other means to help using the formulation most suitably for the treatment. Such measures make a patient pack specifically suitable for and adapted for use for treatment with the aronia extract of the present invention.

The aronia extract may be contained in any appropriate amount in any suitable carrier substance, and it may be present in an amount of 1-99% by weight of the total weight of the composition. The composition may be provided in a dosage form that is suitable for the oral, parenteral (e.g., intravenously, intramuscularly), cutaneous, or skin (patch) administration route. Thus, the composition may be in the form of, e.g., tablets, capsules, pills, powders, granulates, suspensions, emulsions, solutions, gels including hydrogels, pastes, ointments, creams, plasters or drenches.

The pharmaceutical compositions may be formulated according to conventional pharmaceutical practice (see, e.g., Remington (2020)).

**Examples**

**Example 1: Preparation of an extract according to the invention**

A concentrated chokeberry (*Aronia melanocarpa*) juice with an anthocyanin content of at least 0.5% at 65 Brix (cyanidin-3-O-glucoside chloride equivalent) was purchased and was then diluted in water to about 25 Brix. Diluted product was contacted with Amberlite™ XAD-7HP resin. Resin was washed using acidified water, removing most of sugars and acids. Bound phenolics were then eluted using an acidified hydro-ethanolic solution. Elution solution was collected and concentrated by evaporation until reaching at least 45% dry matter. The concentrated elution solution was then vacuum dried at a temperature between 68°C-70°C and grinded (sieving to less than 500 μm). A red - violet to dark red powder was obtained.

**Example 2: Analysis of an extract according to the invention and of four commercially available extracts**

A dry extract of chokeberry was prepared as described in Example 1 ("DF Extract").

Four commercially available powdered *Aronia melanocarpa* extracts were purchased: Aronox provided by the company Naturex ("Commercial extract 1"), an extract provided by the company Bestgrand Biotech ("Commercial extract 2"); Brainberry provided by the company Bioactor ("Commercial extract 3"); and Aroniacraft provided by the company Iprona ("Commercial extract 4"). All the four commercial extracts had a violet or dark red to dark purple color.

The Table 1 below shows the main features of these powders.

**Table 1**

| | DF Extract | Commercial extracts | | | |
|---|---|---|---|---|---|
| | | 1 | 2 | 3 | 4 |
| Fineness | + | - | + | + | + |
| Color | red - violet to dark red | Dark red to dark purple | Violet | Dark red to dark purple | intensive dark red |
| Flavor | Char. | Char. | Char. odor and taste | Char. flavor | Weak, astringent |
| Solubility | $H_2O$ | $H_2O$ | $H_2O$ | $H_2O$ | $H_2O$ and $H_2O$-EtOH |
| Loss on drying | < 5% | < 8% | ≤ 8% | < 5% | 2.40% |
| Particle size* | 500 μm | 420 μm | 177 μm | NA | 250 μm |
| Bulk density (g/mL) | > 0.60 | 0.30 - 0.70 | 0.30 - 0.70 | NA | NA |

Fineness: "+" is for "fine powder", "-" is for "powder"; Flavor: "Char." means "characteristic" * particle size means that more than 95% of the particles pass through the corresponding value.

Experimental procedure

The five samples were analyzed as described below.

*Total polyphenols content*

Samples were analyzed using Folin Ciocalteu Method with a protocol based on the procedure ISO 14502-1:2005. This analytical method is based on the reduction of tungsten and molybdenum oxides by reaction with the phenolics present in vegetables. Oxidized forms of the metal oxides are colorless in the reaction middle and become blue upon the reduction reaction. The blue coloration produced is proportional to the phenolic content in the sample. This coloration is measured by recording the OD at 765 nm. Results are expressed in epicatechin equivalent.

*Anthocyanins content*

Samples were analyzed using a spectrophotometric method procedure for the quantitative determination of the anthocyanins, with a protocol adapted from European Pharmacopoeia 5.0 (01/2005:1602). Samples were diluted into a methanol solution acidified and optical density was read at the appropriate wavelength 528 nm. Results are expressed in Cyanidin-3-O-glucoside chloride equivalent.

*Proanthocyanidins content*

Samples were analyzed using a procedure adapted from Prior et al. (2010). This colorimetric assay is based on the regiospecific reaction of DMAC (p-dimethylaminocinnamaldehyde) with the phenolic function on terminal unit of the PACs polymers. The reaction products are green and are detected by spectrophotometry at 640 nm. Quantification was done by comparison to the external calibration curve built for procyanidin A2. Results are expressed in procyanidin A2 equivalent.

*Color parameters*

The color parameters were measured at constant anthocyanins content. A buffer at pH=3 was prepared by admixing demineralized water with trisodium citrate, citric acid and potassium sorbate. All the samples were diluted in the buffer, until an anthocyanins concentration of 0.04 g/L was reached. Measures were made using a Minolta spectrocolorimeter. The L*a*b* is based on the appreciation of the shades of color of a product by analyzing its spectral curve with a spectrocolorimeter equipped with an integrating sphere. The color space used is characterized by three values:

- L*: clarity, axial axis ranging from 0 (opacity) to 100 (transparency)
- a*: hue or chromatocity, horizontal axis from +60 (red) to -60 (green)
- b*: hue or chromatocity, vertical axis from +60 (yellow) to -60 (blue).

Two other values can be measured:

- C*: saturation
- H: tint angle.

<u>Results</u>

The results are summarized in Table 2.

*Total polyphenols content*

The "Extract DF" had a higher total polyphenol content as compared to the 4 commercial extracts analyzed. The polyphenol content of the "Extract DF" was higher than 60 dry wt% of the extract, whereas it was below 50 dry wt% for all the commercial extracts 1 to 4.

*Anthocyanin content*

The anthocyanin content of the "Extract DF" was in the range of 11 to 15 dry wt%, whereas the commercial extracts 1 and 4 had less than 10 dry wt% of anthocyanins, and the commercial extracts 2 and 3 had more than 20 dry wt%.

*Proanthocyanidin content*

The "Extract DF" had the highest content of proanthocyanidins, corresponding to more than 20 dry wt%. The proanthocyanidin content was of 18.5 dry wt% for the Commercial extract 2, and of less than 10 dry wt% for the Commercial extracts 1, 3 and 4.

*Ratio proanthocyanidins / anthocyanins*

The proanthocyanidins / anthocyanins ratio of Extract DF is of about 2.0. This value is 2 to 10 times higher than that measured for the Commercial extracts (i.e. between 0.2 and 1.0).

*Color parameters*

The values are presented in Table 2 and Figure 1. These results confirm what was observed to the naked eye The "Extract DF" has specific color parameters which differentiate it from the Commercial extracts. The delta E*ab is a standard measure to assess the difference in color between several samples. It is considered significant when its value is higher than 2. The calculated delta E*ab between the Extract DF on one hand and the Commercial extracts 1 to 4 on the other hand was of 10, 13, 17 and 10 respectively, confirming that the Extract DF is visually significantly different than the other. Namely, Extract DF is less red/green, less yellow/blue, bluer, less orange and less shiny than the Commercial extracts 1 to 4.

**Table 2: comparison between the extract of the invention and commercial extracts.**

| | Extract DF | Commercial extracts | | | |
|---|---|---|---|---|---|
| Parameters | | 1 | 2 | 3 | 4 |
| (i) Total polyphenols | 62 | <50 | <50 | <40 | <15 |
| (ii) Anthocyanins | 12.6 | <10 | >24 | >25 | <10 |
| (iii) Proanthocyanidins | 25 | <10 | <19 | <10 | <2 |
| Ratio (iii)/(ii) | 2.0 | 1.0 | 0.7 | 0.3 | 0.2 |
| L* | 68.48 | 65.81 | 67.14 | 68.1 | 69.92 |
| a* | 51.01 | 56.56 | 58.7 | 60.09 | 56.77 |
| b* | 13.33 | 21.16 | 23.22 | 27.56 | 21.2 |
| c* | 52.72 | 60.39 | 63.13 | 66.11 | 60.6 |
| h* | 14.65 | 20.51 | 21.58 | 24.64 | 20.48 |

Contents (i), (ii) and (iii) are expressed in dry wt % of the total dry weight of the extract.

**Example 3: Antioxidant effects**

The functional antioxidant properties of the aronia extracts "DF extract" and the commercial extracts No. 1 to 3 were investigated.

Direct (i.e. intracellular) antioxidant effect of each aronia extract was determined in relation to HepG2 cell line model using innovative cell-based assays for testing for intracellular radical ROS scavenging activity with AOP1 ("Anti Oxidant Power 1"), and for catalase-like activity with AOPCAT. AOP1 bioassay measures the ability of an antioxidant to neutralize oxidative stress and the effect is measured by a delay in the kinetic evolution of fluorescence emission (Gironde et al. 2020). AOP CAT is a patented technology (EP3044569). In the present analysis, it is based on intracellular presence of a nucleic acid biosensor whose fluorescence increases with $H_2O_2$. This bioassay measures the ability of an antioxidant to neutralize $H_2O_2$ (dismutation in $O_2$ and $H_2O$) and the effect is measured by a delay in the kinetic evolution of fluorescence emission.

Method

*Cell culture of HepG2 cell lines*

HepG2 (passage 5 to 30) cells were cultured at 37°C in 5% $CO_2$ in Glutamax DMEM medium complemented with 10% FBS and 1X pen-strep solution. Cells were grown up to 70-80% confluence then transferred in clear bottom 96-well microplates for 24h at a density of 106 cells/mL (75$\mu$L, 75000 cells/well).

*Determination of intracellular radical scavenging activity: AOP1 bioassay*

Aronia samples (500 mg) were solubilized at a final concentration of 50 mg/mL in the corresponding cell culture medium. Solutions were centrifuged at 8700 rpm for 10 minutes and experiments were performed using the supernatants. Cells were incubated with samples (9 different concentrations obtained by serial dilutions) for 4 hours at 37 °C in 5% CO2.

After the 4 hours of incubation with samples, cells were treated with thiazole orange (TO) as fluorescent biosensor for 1 hour. Fluorescence (Relative Fluorescence Unit (RFU) at 535 nm) was measured on a Varioskan equipment (Thermo Fisher Scientific, Waltham, MA USA) according to a recurrent 480 nm LED application procedure (20 iterations) of the whole 96-well plate. Raw-data kinetic profiles were recorded, and dose-response curves were modelized using Prism8 software (GraphPad, San Diego, CA, USA). The antioxidant cell index (AOP index) was calculated from normalized kinetic profiles according to the formula:

$$AOP\ index\ (\%) = 100 - 100\ (_0\!\int^{20} RFUsample\ /\ _0\!\int^{20} RFUcontrol)$$

Dose-response curves were obtained by compiling AOP indices according to Log of the sample concentration, and were submitted to a sigmoid fit according to the formula:

$$AOP\ index = AOP\ index_{min} + (AOP\ index_{max} - AOP\ index_{min})\ /\ (1 + 10(Log(EC50-SC)*HS))$$

where SC=sample concentration and HS=Hill slope or tangent slope at inflexion point, to calculate EC50. Experiments were done in cell culture medium without FBS and at least two independent experiments were performed, each on triplicate wells.

*Determination of Catalase-like activity: AOP CAT bioassay*

Aronia samples (at 16 different concentrations obtained by serial dilutions) were incubated with $H_2O_2$ (0.34%) for 1 hour, and experiments were performed using the incubation compounds. The incubation compounds were added to HepG2 cells (14.7% of final volume) which were preliminarily incubated with thiazole orange (TO) as biosensor for 30 min at 37 °C in 5% $CO_2$. Fluorescence (Relative Fluorescence Unit (RFU) at 535 nm) was recorded on a kinetic mode for 75 min, with T=0 as the time of biosensor addition. Bovine liver catalase was used as positive control. Dose-response curves were modelized according to the formula:

$$CAT\text{-}like\ index = 100\ x\ [(_{35}\!\int^{75} RFU\ compound - _{35}\!\int^{75} RFUC\ control)\ /\ (_{35}\!\int^{75} RFU\ SAMPLE\ MAX$$

$$control - _{35}\!\int^{75} RFUC\ control)]$$

EC50 values were calculated from the sigmoid fit according to the formula:

$$Fold\ Increase\ FI = FImin + (FImax - FImin)/(1 + 10(Log(EC50-SC)*HS))$$

where SC=sample concentration and HS=Hill slope. Two independent experiments were performed, each on triplicate wells.

*Statistical analysis*

Analysis was made using Prism8 software (GraphPad, San Diego, CA, USA). For fluorescence profiles, bars corresponded to standard error values obtained from triplicates. For dose-response figures, asymmetrical confidence intervals were reported from Prism. The two experiments that showed the best coefficient of determination ($R^2$) from the nonlinear regression model were used for EC values.

Results

The dose-response curves of each sample are shown in Figure 2.
The EC50 obtained for the DF extract is used as reference value (100%). Table 3 shows the EC50 values and the associated 95% confident interval, and the relative antioxidant effect of each aronia extract tested.
As EC50 is the concentration calculated to obtain 50% of the maximum effect, the lowest the value, the better the effect. As shown in Table 3, the DF extract has the lowest EC50 value in AOP1 and AOPCAT assays, meaning that a lower quantity of DF extract is required to obtain 50% of the maximum effect. This difference is considered statistically significant if the 95% confident interval of EC50 do not overlap. The relative antioxidant effect values of the Commercial extracts were all below 100%, meaning that these products have a lower antioxidative effect vs aronia extract. In AOP1 assays, the relative antioxidant effect of DF extract (100%) was higher than that of commercial extract 1 (83.17%) and commercial extract 3 (84.24%), and significantly higher than that of commercial extract 2 (72.69%). In AOPCAT, the antioxidant effect of the DF extract (100%) was significantly higher and at least twice as that of commercial extract 1 (48.17%), commercial extract 2 (35.85%) and commercial extract 3 (36.78%).

**Table 3: EC50 and relative antioxidant effect in HepG2 cells of DF Extract (cf. Example 1) and Commercial extracts 1 to 3 (cf. Example 2).**

| Compound | | AOP1 | | | AOPCAT | | |
|---|---|---|---|---|---|---|---|
| | | EC50 (mg/mL) | 95% confident interval of EC50 | Relative antioxidant effect (%) | EC50 (mg/mL) | 95% confident interval of EC50 | Relative antioxidant effect (%) |
| DF Extract | | 37.9 | [34.9, 41.15] | 100 | 425.5 | [297.4, 640.2] | 100 |
| Commercial extracts No. | 1 | 45.57 | [38.2, 54.4] | 83.17 | 883.4 | [789, 990] | 48.17 |
| | 2 | 52.14 | [41.3, 65.9] | 72.69 | 1187 | [874, 1612] | 35.85 |
| | 3 | 44.99 | [37.7, 53.7] | 84.24 | 1157 | [903, 1537] | 36.78 |

These EC50 and relative antioxidant effects have been represented as a function of total polyphenol content, of anthocyanin content, of PACs content and of the PACs/anthocyanins ratio in figures 3 (AOP1 assays) and in figure 4 (AOPCAT assays).

Total polyphenol content was slightly correlated to the antioxidant effect, whereas the anthocyanin content was surprisingly not correlated to direct (intracellular) antioxidant effect. At the opposite, PACs appears to be a main component of the direct antioxidant effect observed in HepG2 cells, as it is positively and strongly correlated to the antioxidant effect measured, more particularly in the AOPCAT assay. This becomes even more striking when the ratio of PACs to anthocyanins is considered. Thus, this specific ratio is likely to confer to the DF extract its higher efficacy.

The examples have shown that: i) an aronia extract according to the invention has specific parameters that makes it different from the known aronia extracts in the marketplace. Indeed, it has a high polyphenol and PACs content, and a PACs/anthocyanins ratio of at least 1.5. Also, it has a different color; ii) an aronia extract according to the invention is particularly effective in reducing oxidative stress, and therefore can be used for preventing, limiting, or reducing the oxidative stress due to the exposure to pollutants, aging and/or exercise, and also in the treatment of a disorder related to cellular oxidative stress, such as gut barrier dysfunction, intestinal inflammation, inflammatory bowel diseases, irritable bowel disease, insulin resistance, type 2 diabetes mellitus, metabolic syndrome, cardiovascular disorders, and neurodegenerative disorders.

**References**

Borowska, S., and M. M. Brzóska. 2016. 'Chokeberries (Aronia melanocarpa) and Their Products as a Possible Means for the Prevention and Treatment of Noncommunicable Diseases and Unfavorable Health Effects Due to Exposure to Xenobiotics', Compr Rev Food Sci Food Saf, 15: 982-1017.

Fereidoon, Shahidi, and Marian Naczk. 1995. "Food phenolics : sources, chemistry, effects, applications." In. Basel: Lancaster.

Forman, H. J., K. J. Davies, and F. Ursini. 2014. 'How do nutritional antioxidants really work: nucleophilic tone and para-hormesis versus free radical scavenging in vivo', Free Radic Biol Med, 66: 24-35.

Fraga, C. G., K. D. Croft, D. O. Kennedy, and F. A. Tomás-Barberán. 2019. 'The effects of polyphenols and other bioactives on human health', Food Funct, 10: 514-28.

Gironde, C., M. Rigal, C. Dufour, and C. Furger. 2020. 'AOP1, a New Live Cell Assay for the Direct and Quantitative Measure of Intracellular Antioxidant Effects', Antioxidants (Basel), 9.

Holmstrom, K. M., and T. Finkel. 2014. 'Cellular mechanisms and physiological consequences of redox-dependent signalling', Nat Rev Mol Cell Biol, 15: 411-21.

Pandey, K. B., and S. I. Rizvi. 2009. 'Plant polyphenols as dietary antioxidants in human health and disease', Oxid Med Cell Longev, 2: 270-8.

Prior, R. L., E. Fan, H. Ji, A. Howell, C. Nio, M. J. Payne, and J. Reed. 2010. 'Multi-laboratory validation of a standard method for quantifying proanthocyanidins in cranberry powders', J Sci Food Agric, 90: 1473-8.

Remington. 2020. Remington: The Science and Practice of Pharmacy

## Claims

1. An aronia extract comprising proanthocyanidins (PACs) and anthocyanins, wherein:

   - the polyphenol content is at least 55 dry wt.%, preferably at least 60 dry wt.%,
   - the PACs content is at least 19 dry wt.%, preferably at least 20 dry wt.%,
   - the PACs:anthocyanins ratio is at least 1.5, and

   wherein the polyphenol content can be measured by the Folin-Ciocalteu (epicatechin equivalent), the proanthocyanidin content can be measured by the BL-DMAC method (procyanidin A2 equivalent), and the anthocyanin content can be measured by spectrophotometry (cyanidin-3-O-glucoside chloride equivalent).

2. The aronia extract of claim 1, wherein:

   - the PACs:anthocyanins ratio is comprised between 1.5 and 4.5, preferably between 1.5 and 3.5, more preferably between 1.5 and 2.5, and even more preferably is of about 2,

3. The aronia extract of claim 1 or 2, wherein:

   - the anthocyanins content is at least 10 dry wt.%, preferably between 10 and 20 dry wt.%, more preferably between 10 and 15 dry wt.%.

4. The aronia extract according to any one of the preceding claims, wherein said Aronia extract is selected from the group consisting of an *Aronia arbutifolia* extract, an *Aronia melanocarpa* extract and an *Aronia prunifolia* extract.

5. The aronia extract according to claim 4, wherein the aronia extract is an extract obtained from the fruits of *Aronia melanocarpa.*

6. The aronia extract according to any of the preceding claims, wherein the extract is in a dry powder form.

7. The aronia extract of claim 6, wherein said extract has the following colour parameters, when measured at 0.04 g/L of anthocyanin in a buffer at a pH value of 3:

   - L* (brightness) between 65 and 70,
   - a* (red/green coordinate) of less than 55, preferably between 45 and 55, more preferably between 50 and 54,
   - b* (yellow/blue coordinate) of less than 20, preferably of less than 15, more preferably between 10 and 15.

8. A process for obtaining the aronia extract according to any one of claims 1 to 7, the process comprising the steps of:

   - selecting a concentrated juice of aronia fruits with an anthocyanin concentration (cyanidin-3-O-glucoside chloride equivalent) of at least 0.5% at 65 Brix,
   - diluting the concentrated juice to about 25 Brix with osmosed water to obtain a diluted juice,
   - contacting, in a chromatographic column, said diluted juice with a macroporous, crosslinked, aliphatic polymer adsorbent resin, wherein the resin has a surface area of at least 300 $m^2$/g, an average pore diameter of about 550 Angstrom, a total pore volume of about 0.5 mL/mL, a water retention capacity of 61 to 69%, said resin being conditioned at a pH equal to or of less than 3.5 with acidified water,
   - washing with acidified water,
   - eluting polyphenols using an acidified hydro-ethanolic solution to obtain an elution solution,
   - optionally, concentrating the elution solution to a dry matter content from 35 to 55% w/w, to obtain a concentrated extract,
   - optionally, drying and grinding said concentrated extract to obtain a dry extract,

   wherein said acidified water is obtained by mixing water with 0.03% (volume/volume) of a hydroxide sulfate solution at a concentration of 96%.

9. The process of claim 8, wherein said concentrated juice of aronia fruits is a concentrated fruit juice obtained from *Aronia melanocarpa.*

10. An aronia extract obtainable by the process according to claim 8 or 9.

11. A food, nutraceutical or cosmetic composition comprising, or consisting of, the aronia extract according to any of the claims 1 to 7, or the aronia extract according to claim 10.

12. Use of a composition according to claim 11 for preventing, limiting, or reducing oxidative stress due to the exposure to pollutants, aging and/or exercise.

13. A pharmaceutical composition comprising the aronia extract according to any of claims 1 to 7, or the aronia extract according to claim 10.

14. The pharmaceutical composition of claim 13 for use in the treatment of a disorder related to cellular oxidative stress in a subject in need thereof.

15. The pharmaceutical composition for use according to claim 14, wherein said disorder is selected from the group consisting of gut barrier dysfunction, intestinal inflammation, inflammatory bowel diseases, irritable bowel disease, insulin resistance, type 2 diabetes mellitus, metabolic syndrome, cardiovascular disorders, and neurodegenerative disorders.

L* Lightness

70

50

30

10

a* Red/Green coordinate

h* Tint angle

b* Yellow/Blue coordinate

c* Saturation

──●──DF extract     ········ Commercial extract 1    ─ ─ ─ Commercial extract 2

──── Commercial extract 3   ─ ─ ─ ─ Commercial extract 4

# FIG. 1

**FIG. 2**

# FIG. 3

FIG. 4

Europäisches
Patentamt

European
Patent Office

Office européen
des brevets

# EUROPEAN SEARCH REPORT

**Application Number**

EP 22 31 5052

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GAO NINGXUAN ET AL: "Optimization of anthocyanidins conversion using chokeberry pomace rich in polymeric proanthocyanidins and cellular antioxidant activity analysis", LWT- FOOD SCIENCE AND TECHNOLOGY, ACADEMIC PRESS, UNITED KINGDOM, vol. 133, 18 July 2020 (2020-07-18), XP086273920, ISSN: 0023-6438, DOI: 10.1016/J.LWT.2020.109889 [retrieved on 2020-07-18] * page 1, right-hand column - page 3, right-hand column * * page 5, left-hand column * * page 7, last paragraph * ----- | 1-7 | INV. A61K36/73 A61P3/10 A23L2/02 A61K31/352 A61P39/06 |
| A | SOSNOWSKA DOROTA ET AL: "Comparison of in vitro anti-lipase and antioxidant activities, and composition of commercial chokeberry juices", EUROPEAN FOOD RESEARCH AND TECHNOLOGY, SPRINGER BERLIN HEIDELBERG, BERLIN/HEIDELBERG, vol. 242, no. 4, 8 October 2015 (2015-10-08), pages 505-515, XP035868081, ISSN: 1438-2377, DOI: 10.1007/S00217-015-2561-4 [retrieved on 2015-10-08] * page 505, right-hand column * * page 509, right-hand column * * page 512 * ----- -/-- | 1-7 | |

**TECHNICAL FIELDS SEARCHED (IPC)**

A61K
A61P
A23L

The present search report has been drawn up for all claims

| Place of search | Date of completion of the search | Examiner |
|---|---|---|
| Munich | 4 August 2022 | Escolar Blasco, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 4**

## EUROPEAN SEARCH REPORT

Europäisches
Patentamt
European
Patent Office
Office européen
des brevets

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SIDOR ET AL: "Black Chokeberry Aronia melanocarpa L.-A Qualitative Composition, Phenolic Profile and Antioxidant Potential", MOLECULES, vol. 24, no. 20, 15 October 2019 (2019-10-15), page 3710, XP055945768, DOI: 10.3390/molecules24203710 * abstract * * page 10; table 2 * * page 50 * | 1-7 | |
| X | KRENN L ET AL: "Anthocyanin- and proanthocyanidin-rich extracts of berries in food supplements – analysis with problems", PHARMAZIE, GOVI VERLAG PHARMAZEUTISCHER VERLAG GMBH, DE, vol. 62, no. 11, 1 November 2007 (2007-11-01), pages 803-812, XP009108561, ISSN: 0031-7144, DOI: 10.1691/PH.2007.11.7621 * page 805; table 1 * * page 806, right-hand column – page 809 * | 1,2,4,5, 7-15 | |

-/--

TECHNICAL FIELDS
SEARCHED        (IPC)

The present search report has been drawn up for all claims

| Place of search | Date of completion of the search | Examiner |
|---|---|---|
| Munich | 4 August 2022 | Escolar Blasco, P |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 22 31 5052

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DENEV PETKO ET AL: "Black chokeberry (Aronia melanocarpa) polyphenols reveal different antioxidant, antimicrobial and neutrophil-modulating activities", FOOD CHEMISTRY, vol. 284, 1 June 2019 (2019-06-01), pages 108-117, XP055948313, NL ISSN: 0308-8146, DOI: 10.1016/j.foodchem.2019.01.108 * page 109 * * page 115, right-hand column, paragraph 3 – page 116, left-hand column, paragraph 1 * * page 111, left-hand column – page 113, left-hand column * | 1,2,4,5, 7-10,13 | |
| X | US 2017/095494 A1 (LEONHART SEBASTIEN [CA] ET AL) 6 April 2017 (2017-04-06) * paragraph [0033] – paragraph [0050]; example 1 * | 8,10,11 | |
| X | US 2012/237649 A1 (DRAVENSTADT LOWELL VERNON [US] ET AL) 20 September 2012 (2012-09-20) * paragraphs [0022], [0031], [0035], [0036] * * paragraphs [0077] – [0079], [0091]; claims; examples 1,2 * | 1-3,6-8, 10,11 | TECHNICAL FIELDS SEARCHED (IPC) |

-/--

The present search report has been drawn up for all claims

| Place of search | Date of completion of the search | Examiner |
|---|---|---|
| Munich | 4 August 2022 | Escolar Blasco, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 4

Europäisches
Patentamt

European
Patent Office

Office européen
des brevets

## EUROPEAN SEARCH REPORT

Application Number

EP 22 31 5052

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BRÄUNLICH MARIE ET AL: "Extracts, Anthocyanins and Procyanidins from Aronia melanocarpa as Radical Scavengers and Enzyme Inhibitors", NUTRIENTS, vol. 5, no. 3, 4 March 2013 (2013-03-04), pages 663-678, XP055945809, DOI: 10.3390/nu5030663 * page 665, paragraph 3 – page 666, paragraph 1 * * page 671 – page 673 * * page 675 * ————— | 1,2,4,5, 7-10, 12-15 | |
| | | | TECHNICAL FIELDS SEARCHED (IPC) |

The present search report has been drawn up for all claims

| Place of search | Date of completion of the search | Examiner |
|---|---|---|
| Munich | 4 August 2022 | Escolar Blasco, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 31 5052

This annex lists the patent family members relating to the patent documents cited in the above-mentioned European search report.
The members are as contained in the European Patent Office EDP file on
The European Patent Office is in no way liable for these particulars which are merely given for the purpose of information.

04-08-2022

| Patent document cited in search report | | Publication date | Patent family member(s) | | Publication date |
|---|---|---|---|---|---|
| US 2017095494 | A1 | 06-04-2017 | CA | 2942777 A1 | 24-09-2015 |
| | | | US | 2017095494 A1 | 06-04-2017 |
| | | | WO | 2015139128 A1 | 24-09-2015 |
| US 2012237649 | A1 | 20-09-2012 | CA | 2758811 A1 | 21-10-2010 |
| | | | EP | 2418970 A1 | 22-02-2012 |
| | | | EP | 2792254 A1 | 22-10-2014 |
| | | | EP | 3132687 A2 | 22-02-2017 |
| | | | ES | 2612343 T3 | 16-05-2017 |
| | | | PL | 2792254 T3 | 30-06-2017 |
| | | | US | 2012237649 A1 | 20-09-2012 |
| | | | US | 2015359253 A1 | 17-12-2015 |
| | | | US | 2016262438 A1 | 15-09-2016 |
| | | | WO | 2010121203 A1 | 21-10-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021122789 A1
- US 20120128800 A1

- EP 3044569 A

### Non-patent literature cited in the description

- **BOROWSKA, S. ; M. M. BRZÓSKA.** Chokeberries (Aronia melanocarpa) and Their Products as a Possible Means for the Prevention and Treatment of Noncommunicable Diseases and Unfavorable Health Effects Due to Exposure to Xenobiotics. *Compr Rev Food Sci Food Saf,* 2016, vol. 15, 982-1017
- **FEREIDOON, SHAHIDI ; MARIAN NACZK.** Food phenolics : sources, chemistry, effects, applications. *Basel: Lancaster,* 1995
- **FORMAN, H. J. ; K. J. DAVIES ; F. URSINI.** How do nutritional antioxidants really work: nucleophilic tone and para-hormesis versus free radical scavenging in vivo. *Free Radic Biol Med,* 2014, vol. 66, 24-35
- **FRAGA, C. G. ; K. D. CROFT ; D. O. KENNEDY ; F. A. TOMÁS-BARBERÁN.** The effects of polyphenols and other bioactives on human health. *Food Funct,* 2019, vol. 10, 514-28

- **GIRONDE, C. ; M. RIGAL ; C. DUFOUR ; C. FURGER.** AOP1, a New Live Cell Assay for the Direct and Quantitative Measure of Intracellular Antioxidant Effects. *Antioxidants (Basel),* 2020, vol. 9
- **HOLMSTROM, K. M. ; T. FINKEL.** Cellular mechanisms and physiological consequences of redox-dependent signalling. *Nat Rev Mol Cell Biol,* 2014, vol. 15, 411-21
- **PANDEY, K. B. ; S. I. RIZVI.** Plant polyphenols as dietary antioxidants in human health and disease. *Oxid Med Cell Longev,* 2009, vol. 2, 270-8
- **PRIOR, R. L. ; E. FAN ; H. JI ; A. HOWELL ; C. NIO ; M. J. PAYNE ; J. REED.** Multi-laboratory validation of a standard method for quantifying proanthocyanidins in cranberry powders. *J Sci Food Agric,* 2010, vol. 90, 1473-8
- **REMINGTON.** Remington: The Science and Practice of Pharmacy. 2020